# EUROPEAN PATENT APPLICATION

(11) **EP 1 047 033 A1**
(43) Date of publication of application: **25.10.2000**
(21) Application number: 00201445.4
(22) Date of filing: 20.04.2000
(51) Int. Cl.: G08B 21/00, A61F 13/42

(54) **Moisture-signalling system for diapers**

(30) Priority: 20.04.1999 NL 1011837
(71) Applicant: N.V. Nederlandsche Apparatenfabriek NEDAP, NL-7141 DE Groenlo (NL)
(72) Inventor: Hogen Esch, Johannes Harm Lukas, 7122 ZN Aalten (NL)
(74) Representative: Prins, Adrianus Willem

(57) **Abstract**

A moisture signalling system for diapers, in which the amount of moisture in the diaper is measured and together with an identification number is radiographically transferred by a measuring clip, to be attached to the diaper, having three tongues between which the front side and rear side of the diaper is fastened. The measurement concerns the electrical resistance of a polymer conductor, which is provided in the diaper from the front to the rear, and whose resistance changes by parallel connection of the absorbed moisture. The conductor is also provided on the upper side of the diaper to make contact with the clip in a simple manner. The initial resistance of the conductor indicates that the clip is correctly attached to the diaper. A disposable bag around the clip prevents skin irritation. The supply of the measuring circuit can be drawn actively from a battery or passively from the transmission field of a transmitter/receiver system.

## Description

This invention relates to a system for in particular remote signalling of moisture in moisture absorbing means such as incontinence diapers, baby diapers, and the like. This invention also relates to a moisture absorbing means, such as a diaper, and a measuring clip of the system.

Such a system is known inter alia from US 5,557,263.

For nurses to check whether incontinence diapers need to be changed is very time-consuming because with the current incontinence diapers this check must be done visually. To that end, these diapers are generally provided with a marking indication with a kind of graduation which discolours or disappears if it comes into contact with urine.

Another known solution to this problem is the so-called pad and buzzer which gives the patient a signal at predetermined time intervals, warning him to go to the toilet.

From the US patent mentioned, a moisture detection system is known in which via a radiographic connection between a sensor attached to the absorbing means and a receiver, a signal is transferred if moisture is detected. Since, due to the use of moisture absorbing materials that form gels upon moisture uptake, modem incontinence diapers are capable of absorbing large amounts of moisture, it is no longer sufficient to signal that there is moisture in the diaper, but it is necessary to know how much moisture has already been absorbed. According as more moisture is absorbed in the diaper, the area where the gel formation occurs will become larger, and the moisture stain will expand. For logistic reasons, therefore, signalling a quantitative threshold in these diapers is insufficient. Moreover, for hygienic reasons, it is not desirable to provide a reusable sensor in the diaper. For this reason, therefore, a communication means that is attached to the diaper needs to be packaged hygienically, or must be used only a single time.

The present invention contemplates providing a solution to these problems and to that end is characterized in that the system comprises an electrical conductor which is provided in the moisture absorbing means, the electrical conductor being provided with a conductor material whose resistance is dependent upon an amount of moisture with which the conductor comes into contact, the measuring signalling system further comprising a measuring clip which, in use, is detachably and conductively connected with the electrical conductor, while the measuring clip comprises an electrical circuit for obtaining information about the magnitude of an electrical resistance of the electrical conductor as measured with the electrical circuit, for obtaining information about the amount of moisture in the absorbing means. According to the invention, information is obtained about the amount of moisture in the absorbing system. Moreover, the measuring clip can be reused.

The moisture absorbing means such as a diaper for infants or an incontinence diaper is characterized in that it is provided with the conductor of the moisture signalling system.

The invention also relates to the measuring clip as such of the moisture signalling system. It is adapted to be connected with the moisture absorbing means, such that the electrical circuit of the measuring clip is electrically connected with the conductor of the moisture absorbing means.

The invention will presently be further elucidated with reference to the drawings. In the drawings:
Fig. 1 schematically shows a diaper provided with a sensor according to the invention;
Fig. 2A shows a measuring clip with which the measured value is transmitted to a receiving system, also shown; and
Fig. 2B shows a side view of the measuring clip according to Fig. 2A

Determining the amount of moisture in a moisture absorbing means, such as a diaper, preferably occurs, according to the invention, by measuring the electrical resistance of a conductor provided in the diaper. This conductor consists of a material of a relatively high electrical resistance. Suitable for this purpose are, for instance, coatings of conductive polymers such as polyaniline and polypyrrole. This coating may be provided on a tape which is secured in the diaper. The coating may also be sprayed via a spray nozzle directly onto the carrier material of the diaper in the form of a narrow track or may optionally even be provided integrally on this carrier material.

Instead of using conductive polymer for the conductor, it is also possible to use a very thin electrically conductive metal or metal oxide layer sputtered onto a tape.

The measurement of the electrical resistance of the conductor is done, according to the invention, with the aid of a measuring clip which is provided with an electrical circuit comprising a measuring circuit and means for transferring the measured value by contactless route to a receiving system which processes the values received, possibly coming from several diapers, and optionally passes a signal to an attendant.

The principle of the measurement according to the invention of the amount of moisture present in the diaper is based on the phenomenon that a part of the electrical resistance of the conductor obtains a low parallel resistance and, as it were, is short-circuited by the moisture present, yielding a decrease of the electrical resistance measured over the conductor. The moisture here forms a resistance which is connected in parallel with (a part of) the conductor material of the conductor, so that the resistance of the conductor is reduced as a result of the moisture. The conductor preferably extends between a front and a rear side of the diaper. To make this reduction of the electrical resistance independent of the position where the moisture is present in the diaper, the electrical resistance is preferably measured over a path from the front to the rear of the diaper.

The place where the front and the rear of the diaper meet and where, accordingly, the measuring clip is to be preferably attached is the portion where the adhesive strips are situated and where the front and rear of the diaper overlap by a small portion. To have greater freedom regarding the position where the clip is to be fitted, the conductor is preferably provided on the upper side, both at the front and the rear, so as to extend horizontally over a portion.

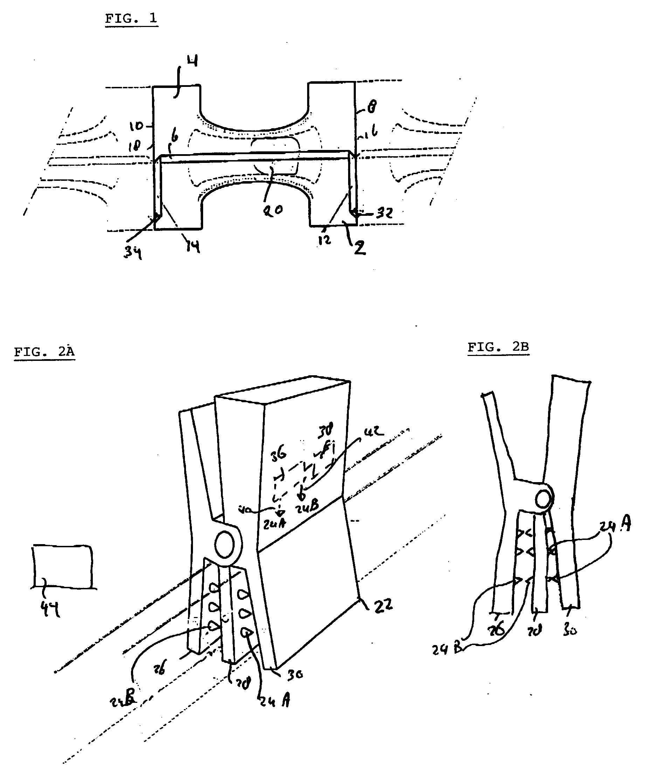

Fig. 1 schematically shows a diaper 2 and the manner in which the diapers are produced while mutually attached. On a carrier foil 4 of the diaper, a conductor 6 is provided, which consists of a tape provided with conductive polymer coating of a relatively high resistance. To obtain a horizontal connecting portion, the conductor 6, both along a front side 8 and along a rear side 10 of the diaper, is laid over a certain portion 12, 14 along an upper side 16, 18 of the diaper. By cutting out the diapers at the end of the production line, there is obtained for each diaper 2 a conductor 6 from the front to the rear, whose total electrical resistance is dependent on the initial electrical resistance of the conductive material of the conductor, measured in dry condition, and the magnitude of a moisture stain 20 present. Obviously, the conductive coating can also be applied directly to the diaper material via one or more spray nozzles or by way of transfer rollers. Also, the conductor 6 can be provided integrally on the carrier foil 4, which, however, necessitates a greater square resistance value of the coating to obtain sufficient measuring accuracy.

The connection of the clip 22 occurs preferably, as indicated in Figs. 2A and 2B, by means of electrical contacts which are made of needle-shaped design, in this example thin needles 24A and 24B, which are provided on at least three guide tongues 26, 28 and 30 of the clip or peg 22. The tongues 26 and 28 form a first pair of tongues and the tongues 28 and 30 form a second pair of tongues. When fitting the diaper, the upper side 18 of the rear side 10 of the diaper 2 with the horizontal portion 14 of the conductor 6 thereon is slipped between the tongues 26 and 28, and the upper side 16 of the front side 8 of the diaper 2 with the likewise horizontal portion 12 of the conductor 6 thereon is slipped between the tongues 28 and 30. The needles 24B, 24A on the back and front of the middle tongue 28, respectively, are electrically insulated from each other and respectively electrically connected with the needles 34B on the tongue 26 and the needles 24A on the tongue 30. A measuring circuit 36 in the clip having attached thereto a contactless radio-frequency communication means 38 in the clip is now connected via conductors 40, 42 in the tongues 26, 28 and 30 with ends 32, 34 of the conductor 6 which is provided in the diaper. Because presently a more or less known electrical resistance value is measured, the measuring circuit 36 in the clip 2 switches on and will, for instance at predetermined time intervals, transmit the measured value of the electrical resistance, or a measuring value related to the electrical resistance value of the conductor 6 measured in dry condition, to a receiving system 44, which converts the received measuring value into a signalling to an attendant. This signalling can, for instance, be done exclusively if a particular moisture threshold quantity is exceeded.

By slightly varying the time intervals between the transmissions of the measuring values, and transmitting together with this measuring value an identity number of the measuring clip as stored in the electrical circuit formed by the measuring circuit 36 and the communication means 38, measuring values of several diapers 2 can be distinguishably transmitted to one and the same receiving system 44. This requires the time intervals for transmission to be relatively long with respect to the time needed for transmitting a single measuring report.

A great advantage of the measuring method according to the invention is further that with the same system it can be determined whether the clip 2 is correctly fitted on the diaper, because if it is improperly fitted, either a short-circuit with too low an electrical resistance, or too high an initial electrical resistance is measured.

From the viewpoint of hygiene, and to prevent skin irritation due to the clip, as well as to enhance wearing convenience, optionally use can be made of a disposable bag in which the clip is placed prior to being fitted, and which is pierced by the needles making contact with the conductor in the diaper.

The communication between the clip 22 and the receiving system 44 can occur actively, with the supply of the measuring circuit in the clip 22 and the transfer to the receiving system 44 proceeding from a small battery. To bridge a largest possible distance, preferably use is made of a relatively high transmission frequency of, for instance, 433 MHz. It is also possible to have the clip 22 function passively, with the energy for the measurement and for the transfer of the measuring value being obtained from an interrogation field of the receiving system 44. To that end, the communication means 38 may be provided with a resonant circuit known per se. Communication between the clip 22 and the receiving system 44 can then take place in a manner based both on the absorption and on the transmission principle. The distance that can then be bridged is small in proportion to the active transfer, and the antenna of a passive clip is more complex in connection with the much lower frequency that is necessary in a passive system.

The moisture signalling system according to the invention for moisture absorbing means such as diapers therefore comprises an electrical conductor 6 which is provided in the moisture absorbing means 2, in this example the diaper. The electrical conductor is provided with a conductive material, while the resistance of the conductor is dependent upon the amount of moisture with which the conductive material comes into contact. The measuring signalling system further comprises the measuring clip 22 which, in use, is detachably and electrically conductively connected with the electrical conductor. The measuring clip 22 comprises an electrical circuit 36, 38 for obtaining, by means of a measurement, information about the magnitude of an electrical resistance of the electrical conductor 6 as measured with the electrical circuit 36, 38, for obtaining information about the amount of moisture in the moisture absorbing means 4. Preferably, the conductor extends between the front side 8 and the rear side 10 of the moisture absorbing means. The conductive material of the conductor 6 has a higher electrical resistance with respect to the electrical resistance of the moisture to be absorbed by the moisture absorbing means, so that the total electrical resistance of the conductor decreases by the connection of the electrical resistance of the moisture absorbed in the absorbing means in parallel with the conductive material. As a result, changes in the electrical resistance of the conductor as measured by the electrical circuit 36, 38 are rendered independent of the position where the moisture is located in the moisture absorbing means. The above-mentioned information about the magnitude of the measured electrical resistance can comprise, for instance, the change of the electrical resistance relative to an initial value of the electrical resistance when the diaper is still dry. (The resistance of the dry conductive material.) However, the above-mentioned information can also comprise the magnitude of the measured electrical resistance as such. The information referred to here is understood to include a quantity, or a change of a quantity, which can be respectively derived from the electrical resistance, or the change of the electrical resistance. Such variants are all understood to fall within the scope of the invention. The moisture signalling system in this example further comprises the receiving system 44, while the electrical circuit 36, 38 of the measuring clip for obtaining the above-mentioned information about the electrical resistance of the conductor is further adapted for the wireless transmission of the information about the measured electrical resistance, and hence about the measured amount of moisture, to the receiving system 44. The receiving system can generate a signal when the measured amount of moisture exceeds a particular threshold value. In particular, it holds that the electrical circuit 36, 38 is further adapted for transmitting an identification code of the measuring clip. In this way, the measuring clip which feeds the above-mentioned information about the amount of moisture in the moisture absorbing means to the receiving system 44 can be identified. In that case, also the absorbing means in question is identified, and so is the person wearing it. An attendant, upon receiving a signal from an identified measuring clip, can proceed to take focused action, for instance by replacing the moisture absorbing means by a clean moisture absorbing means.

The receiving system 44 in this example is further adapted for generating an electromagnetic interrogation field. The electrical circuit 36, 38 of the measuring clip in this example is preferably designed as a passive circuit with a resonant circuit that responds when this circuit is introduced into the electromagnetic interrogation field. Thus the electrical circuit draws energy from the electromagnetic interrogation field in order to function. In that case, no battery is needed, as is the case in an active system.

As mentioned, the electrical circuit can also be designed as an active circuit with a battery. The advantage of this is that the distance between the measuring clip and the receiving system 44 can be greater than when the measuring clip is provided with a passive electrical circuit.

In particular, it holds that the electrical circuit 36, 38 of the measuring clip is adapted for periodically transferring information about the measured amount of moisture. This can be carried out, for instance, once every 15 minutes.

In particular, it holds that the measuring clip comprises at least a first and a second pair of tongues 26, 28, the first pair of tongues being adapted, in use, to encompass a portion of a front side 8 of the absorbing means which, for instance, is in overlap with a rear side 10 of the absorbing means. The second pair of tongues 28, 30 is adapted, for instance, to encompass, in use, a portion of the rear side 10 of the absorbing means which, for instance, is in overlap with the above-mentioned front side of the absorbing means. The tongues are then preferably provided with the electrical contacts which, for instance, are of needle-shaped design, and project from the tongues and which connect the electrical conductor 6, in use, electrically with the electrical circuit 36, 38 of the measuring clip. The clip 22 can also be designed such that the first pair of tongues make contact with a first portion of the conductor 6, while the second pair of tongues make contact with a second portion of the conductor 6, which portions are different from each other.

In particular, and preferably, these portions are the respective ends 32, 34 of the conductor 6. However, this is not requisite. In the latter case, the clip 22 can be designed such that the first and the second pair of tongues have one tongue in common. In fact, in that case, in use, the ends 32 and 34 of the conductor overlie each other (and the above-mentioned portions of the front and rear side of the moisture absorbing means overlap) and can therefore be connected with each other by means of a clip with three tongues located above each other, as shown in Fig. 2B. The first pair of tongues and the second pair of tongues may also comprise no common tongue, in which case it is possible, in principle, to connect, besides the above-mentioned end 32 and 34, other mutually different parts of the conductor with the measuring circuit 36.

Preferably, the moisture signalling system is adapted to check whether the measuring clip is properly electrically connected with the conductor. This can be done by measuring the initial resistance of the conductor (this is the dry resistance of the conductive material if the measuring clip is correctly connected) by means of the measuring clip. Measuring the initial resistance and deciding whether it is correct can be carried out by the electrical circuit 36, 38. The moisture signalling system can be adapted for initialising the measurement of the amount of moisture when the measured initial resistance agrees within predetermined limits with the expected initial value. Determining whether a measured initial resistance is within the predetermined limits can be carried out both by the electrical circuit 36, 38 itself and by the receiving device 44.

Preferably, the measuring clip is included in a replaceable bag of, for instance, plastic.

In that case, the above-mentioned electrical contacts, i.e. the needles 24A, 24B will have been caused to pierce the bag in question. When the moisture absorbing means, in this example the diaper, is to be replaced, the measuring clip can be removed together with the bag. Thereafter the bag is discarded and a new bag is fitted around the measuring clip. The above-mentioned needles are then caused to pierce the bag again. Thereafter a person is provided with a clean moisture absorbing means. After this moisture absorbing means, in particular the diaper, has been fitted, the measuring clip with the bag can be fastened to the moisture absorbing means, such that the electrical circuit 36, 38 of the clip is connected with the conductor 6 of the newly fitted clean moisture absorbing means.

The conductor can comprise, for instance, a tape, which is provided with an electrically conductive polymer. It is also possible, however, that the conductor is provided with a coating of electrically conductive polymer which has been sprayed as a track in the moisture absorbing means. Further, it is possible that the conductor is provided with a tape having sputtered thereon a thin electrically conductive metal or metal oxide layer. As shown in Fig. 1, further, the conductor preferably extends along an upper side 18 of a rear side 10, from the rear side 10 to the front side 8, and along an upper side 16 of the front side 8 of the moisture absorbing means. It is also conceivable that the conductor is provided with other conductive materials, for instance conductive materials whose resistance is dependent in any other way on the amount of moisture with which is comes in contact. The electrical circuit 36, 38 and/or the receiving means 44 can then be adjusted accordingly. Such variants are all understood to fall within the scope of the invention.

## Claims

1. A moisture signalling system for in particular remote signalling of moisture in moisture absorbing means such as diapers, characterized in that the system comprises an electrical conductor which is provided in the moisture absorbing means, the electrical conductor being provided with a conductor material, the resistance of the conductor being dependent on an amount of moisture with which the conductor material comes into contact, the moisture signalling system further comprising a measuring clip which, in use, is detachably and electrically conductively connected with the conductor, while the measuring clip comprises an electrical circuit for obtaining information, by means of a measurement, about the magnitude of an electrical resistance of the electrical conductor, for obtaining information about the amount of moisture in the absorbing means.

2. A moisture signalling system according to claim 1, characterized in that the conductor extends between a front side and a rear side of the moisture absorbing means.

3. A moisture signalling system according to claim 2, characterized in that the conductor material has a high electrical resistance relative to the electrical resistance of the moisture to be absorbed by the moisture absorbing means, so that the total electrical resistance of the conductor decreases through parallel connection of the electrical resistance of moisture absorbed in the moisture absorbing means with the conductor material, so that the change in the electrical resistance of the conductor as measured by the electrical circuit is independent of the position where the moisture is located in the moisture absorbing means.

4. A moisture signalling system according to any one of the preceding claims, characterized in that said information about the magnitude of the measured electrical resistance comprises the change of the electrical resistance relative to an initial value of the electrical resistance.

5. A moisture signalling system according to any one of the preceding claims, characterized in that the system further comprises a receiving system, while the electrical circuit of the measuring clip for obtaining the information about the electrical resistance of the conductor is further adapted for wireless transmission of this information about the measured electrical resistance, and hence about the measured amount of moisture, to the receiving system.

6. A moisture signalling system according to claim 5, characterized in that the receiving system generates a signal when the measured amount of moisture exceeds a threshold value.

7. A moisture signalling system according to claim 5 or 6, characterized in that the electrical circuit is further adapted for transmitting an identification code of the measuring clip to the receiving system.

8. A moisture signalling system according to any one of claims 5-7, characterized in that the receiving system is further adapted for generating an electromagnetic interrogation field, while the electrical circuit of the measuring clip is designed as a passive circuit having a resonant circuit which responds when this circuit is introduced into the electromagnetic interrogation field.

9. A moisture signalling system according to any one of claims 5-7, characterized in that the electrical circuit is designed as an active circuit with a battery.

10. A moisture signalling system according to any one of claims 5-9, characterized in that the electrical circuit of the measuring clip is adapted for periodically transferring the information obtained by means of the electrical circuit.

11. A moisture signalling system according to any one of the preceding claims, characterized in that the measuring clip comprises at least a first and second pair of tongues, the first pair of tongues being adapted, in use, to encompass a portion of a front side of the moisture absorbing means, and the second pair of tongues being adapted, in use, to encompass a portion of a rear side of the absorbing means, while the tongues are provided with electrical contacts which connect the electrical conductor electrically with the electrical circuit of the measuring clip.

12. A moisture signalling system according to claim 11, characterized in that the electrical contacts are of needle-shaped design and project from the tongue.

13. A moisture signalling system according to claim 2 and according to claims 11 or 12, characterized in that the first and second pair of tongues comprise a common tongue which is located between the other tongues of the first and second pair, for respectively comprising the portion of the front side and the portion of the rear side, while these portions, in use, are in mutual overlap.

14. A moisture signalling system according to any one of the preceding claims, characterized in that the moisture signalling system is adapted for checking whether the measuring clip is properly electrically connected with the conductor by measuring the initial resistance of the conductor by means of the measuring clip.

15. A moisture signalling system according to claims 14, characterized in that the moisture signalling system is adapted for switching on the electrical circuit for measuring the amount of moisture when the measured initial resistance agrees within predetermined limits with an expected initial resistance.

16. A moisture signalling system according to any one of the preceding claims, characterized in that the measuring clip is included in a replaceable bag of, for instance, plastic.

17. A moisture signalling system according to claims 12 and 16, characterized in that the electrical contacts have been caused to pierce the bag.

18. A moisture signalling system according to any one of the preceding claims, characterized in that the conductor comprises a tape which is provided with an electrically conductive polymer.

19. A moisture signalling system according to any one of the preceding claims 1-17, characterized in that the conductor is provided with a coating of an electrically conductive polymer which has been sprayed as a track in the moisture absorbing means.

20. A moisture signalling system according to any one of claims 1-17, characterized in that the conductor is provided with a tape having sputtered thereon a thin electrically conductive metal or metal oxide layer.

21. A moisture signalling system according to any one of the preceding claims, characterized in that the conductor respectively extends along an upper side of a rear side, from the rear side to a front side, and along an upper side on the front side of the moisture absorbing means.

22. A moisture absorbing means, such as a diaper for infants or an incontinence diaper, provided with an electrical conductor of the moisture signalling system according to any one of the preceding claims.

23. A moisture absorbing means according to claim 22, characterized in that the conductor extends between a front side and a rear side of the moisture absorbing means.

24. A moisture absorbing means according to claim 23, characterized in that a conductor material of the conductor has a high electrical resistance relative to the electrical resistance of the moisture to be absorbed by the absorbing means, so that the total electrical resistance of the conductor decreases by parallel connection of the electrical resistance of moisture absorbed in the absorbing means and the conductor material.

25. A moisture absorbing means according to any one of the preceding claims 22-24, characterized in that the conductor comprises a tape which is provided with an electrically conductive polymer.

26. A moisture absorbing means according to any one of the preceding claims 22-24, characterized in that the conductor is provided with a coating of electrically conductive polymer which has been sprayed as a track in the moisture absorbing means.

27. A moisture absorbing means according to any one of claims 22-24, characterized in that the conductor is provided with a tape having sputtered thereon a thin electrically conductive metal or metal oxide layer.

28. A moisture absorbing means according to any one of the preceding claims 22-27, characterized in that the conductor respectively extends along an upper side of a rear side, from the rear side to a front side, and along an upper side on the front side of the moisture absorbing means.

29. A measuring clip of the moisture signalling system according to any one of the preceding claims 1-21.

30. A measuring clip according to claim 29, characterized in that the measuring clip is adapted to be connected with a moisture absorbing means, such that an electrical circuit of the measuring clip is electrically connected with an electrical conductor of the moisture absorbing means which extends from a front side to a rear side of the moisture absorbing means.

31. A measuring clip according to claim 30, characterized in that the measuring clip is provided with at least a first and second pair of tongues, the first pair of tongues being adapted, in use, to encompass a portion of a front side of the moisture absorbing means and the second pair of tongues being adapted, in use, to encompass a portion of a rear side of the moisture absorbing means, the tongues being provided with electrical contacts which connect the conductor of the moisture absorbing means electrically with the electrical circuit of the measuring clip.

32. A measuring clip according to claim 31, characterized in that the electrical contacts are of needle-shaped design and project from the tongue.

33. A measuring clip according to claim 31 or 32, characterized in that the first and second pair of tongues comprise a common tongue which is located between the other tongues of the first and second pair.

34. A measuring clip according to any one of claims 30-33, characterized in that the electrical circuit is adapted for obtaining, by means of a measurement, information about the magnitude of an electrical resistance of the electrical conductor for obtaining information about the amount of moisture in the absorbing means.

35. A measuring clip according to claim 34, characterized in that the electrical circuit of the measuring clip for obtaining the information about the electrical resistance of the conductor is further adapted for the wireless transmission of this information to a receiving system.
